# EUROPEAN PATENT APPLICATION

(11) **EP 3 111 826 A1**
(43) Date of publication of application: **04.01.2017**
(21) Application number: 15882016.7
(22) Date of filing: 16.09.2015
(51) Int. Cl.: A61B 1/12, G02B 23/24

(54) **ENDOSCOPE REPROCESSOR**

(30) Priority: 09.02.2015 JP 2015023439
(71) Applicant: OLYMPUS CORPORATION, Hachioji-shi Tokyo 192-8507 (JP)
(72) Inventor: TAKADA Hiroo, Tokyo 192-8507 (JP)
(74) Representative: Schicker, Silvia
(86) International application number: PCT/JP2015/076296
(87) International publication number: WO 2016/129142

(57) **Abstract**

An endoscope reprocessor includes a treatment tank configured to accommodate an endoscope, a treatment tank temperature measuring section configured to measure a temperature of a liquid in the treatment tank, a medicinal solution tank configured to store a medicinal solution, a heating section configured to heat the medicinal solution inside the medicinal solution tank, a first transfer section configured to transfer a part of the medicinal solution to the treatment tank from the medicinal solution tank, a second transfer section configured to transfer the medicinal solution in the treatment tank to the medicinal solution tank, and a control unit configured to replace at least a part of the medicinal solution stored in the treatment tank with the medicinal solution remaining in the medicinal solution tank by controlling the first transfer section and the second transfer section when a measurement result by the treatment tank temperature measuring section is lower than a predetermined threshold value.

## Description

### Technical Field

The present invention relates to an endoscope reprocessor which brings an endoscope into contact with a medicinal solution.

### Background Art

Reprocessing using a medicinal solution such as a cleaning solution and a disinfecting solution is applied to the endoscopes which are used in the medical field, after use. For example, Japanese Patent Application Laid-Open Publication No. 11-76143 discloses an endoscope cleaning apparatus that automatically applies reprocessing using a medicinal solution to an endoscope which is disposed in a treatment tank.

In medicinal solutions for applying reprocessing to endoscopes, there are some medicinal solutions which need to have a temperature of a predetermined value or more to exhibit effects sufficiently. For example, in the endoscope cleaning apparatus which is disclosed in Japanese Patent Application Laid-Open Publication No. 11-76143, a heating section that heats a medicinal solution is provided in a medicinal solution tank which stores the medicinal solution.

When a medicinal solution is heated in a medicinal solution tank as in the endoscope cleaning apparatus which is disclosed in Japanese Patent Application Laid-Open Publication No. 11-76143, for example, if the temperature of the wall face of a treatment tank is lower than the temperature of the medicinal solution, the temperature of the medicinal solution is likely to be reduced by the medicinal solution being transferred into the treatment tank from the medicinal solution tank. When the temperature of the medicinal solution becomes lower than a predetermined value in the treatment tank, a standby time for heating the medicinal solution again by the heating portion section occurs, and a time period which is required to apply cleaning processing to the endoscope becomes long.

The present invention solves the aforementioned problem, and has an object to provide an endoscope reprocessor capable of reducing a time period which is required to carry out reprocessing.

### Disclosure of Invention

### Means for Solving the Problem

An endoscope reprocessor according to one aspect of the present invention includes a treatment tank configured to accommodate an endoscope to cause the endoscope to contact a medicinal solution, a treatment tank temperature measuring section configured to measure a temperature of the medicinal solution stored in the treatment tank, a medicinal solution tank configured to have a larger storage capacity for the medicinal solution than the treatment tank, a heating section configured to heat the medicinal solution inside the medicinal solution tank, a first transfer section configured to transfer a part of the medicinal solution heated by the heating section to the treatment tank from the medicinal solution tank, a second transfer section configured to transfer the medicinal solution of the treatment tank to the medicinal solution tank, and a control unit configured to be connected to the treatment tank temperature measuring section, the first transfer section and the second transfer section, and replace at least a part of the medicinal solution stored in the treatment tank with the medicinal solution remaining in the medicinal solution tank by controlling the first transfer section and the second transfer section, when a measurement result by the treatment tank temperature measuring section is lower than a predetermined threshold value.

Further, an endoscope reprocessor according to another aspect of the present invention includes a treatment tank configured to accommodate an endoscope to cause the endoscope to contact a medicinal solution, a medicinal solution tank configured to have a larger storage capacity for the medicinal solution than the treatment tank, a medicinal solution tank temperature measuring section configured to measure a temperature of the medicinal solution stored in the medicinal solution tank, a heating section configured to heat the medicinal solution inside the medicinal solution tank, a first transfer section configured to transfer a part of the medicinal solution heated by the heating section to the treatment tank from the medicinal solution tank, a second transfer section configured to transfer the medicinal solution of the treatment tank to the medicinal solution tank, and a control unit configured to be connected to the medicinal solution tank temperature measuring section, the first transfer section and the second transfer section, and cause the medicinal solution to circulate between the treatment tank and the medicinal solution tank by controlling the first transfer section and the second transfer section, until a measurement result by the medicinal solution tank temperature measuring section does not change for a predetermined time period.

Further, an endoscope reprocessor according to another aspect of the present invention includes a treatment tank configured to accommodate an endoscope to cause the endoscope to contact a medicinal solution, a treatment tank temperature measuring section configured to measure a temperature of the medicinal solution stored in the treatment tank, a medicinal solution tank configured to have a larger storage capacity for the medicinal solution than the treatment tank, a medicinal solution tank temperature measuring section configured to measure a temperature of the medicinal solution stored in the medicinal solution tank, a heating section configured to heat the medicinal solution inside the medicinal solution tank, a first transfer section configured to transfer a part of the medicinal solution heated by the heating section to the treatment tank from the medicinal solution tank, a second transfer section configured to transfer the medicinal solution of the treatment tank to the medicinal solution tank, and a control unit configured to be connected to the treatment tank temperature measuring section, the medicinal solution tank temperature measuring section, the first transfer section and the second transfer section, and cause the medicinal solution to circulate between the treatment tank and the medicinal solution tank by controlling the first transfer section and the second transfer section, until a temperature difference between the treatment tank and the medicinal solution tank stays in a predetermined range.

### Brief Description of the Drawings

Fig. 1 is a diagram showing a schematic configuration of an endoscope reprocessor of a first embodiment;
Fig. 2 is a flowchart of medicinal solution introduction processing of the endoscope reprocessor of the first embodiment;
Fig. 3 is a diagram showing a state where a medicinal solution of a predetermined volume is introduced into a treatment tank, in the medicinal solution introduction processing;
Fig. 4 is a flowchart showing a modification of the medicinal solution introduction processing of the endoscope reprocessor of the first embodiment;
Fig. 5 is a diagram showing a schematic configuration of an endoscope reprocessor of a second embodiment;
Fig. 6 is a flowchart of medicinal solution introduction processing of the endoscope reprocessor of the second embodiment;
Fig. 7 is a flowchart of medicinal solution introduction processing of an endoscope reprocessor of a third embodiment;
Fig. 8 is a diagram showing a schematic configuration of an endoscope reprocessor of a fourth embodiment; and
Fig. 9 is a flowchart of the medicinal solution introduction processing of the endoscope reprocessor of the fourth embodiment.

### Best Mode for Carrying Out the Invention

Hereinafter, preferred embodiments of the present invention will be described with reference to the drawings. In the respective drawings which will be used in the following explanation, reduced scales are caused to differ according to the respective components, in order to make the respective components have enough sizes to be recognized in the drawings, and the present invention is not limited only to numbers and quantities of the components, shapes of the components, ratios of the sizes of the components and relative positional relationships of the respective components, which are described in the drawings.

Note that in the following explanation, an upper part refers to a position farther away from the ground with respect to an object to be compared, and a lower part refers to a position closer to the ground with respect to an object to be compared. Further, high and low in the following explanation indicate a height relation along a gravitational direction.

### (First embodiment)

Hereinafter, an example of the embodiment of the present invention will be described. An endoscope reprocessor 1 is an apparatus configured to apply reprocessing using a medicinal solution such as a cleaning solution and a disinfecting solution to at least one of an endoscope 50 and an accessory 52 of the endoscope 50. Regeneration treatment mentioned here is not specially limited, and may be any of rinse treatment by water, cleaning treatment that removes contamination such as organic matter, disinfecting treatment that nullifies predetermined microorganisms, sterilization treatment that eliminates or annihilates all microorganisms, or combinations of the treatments.

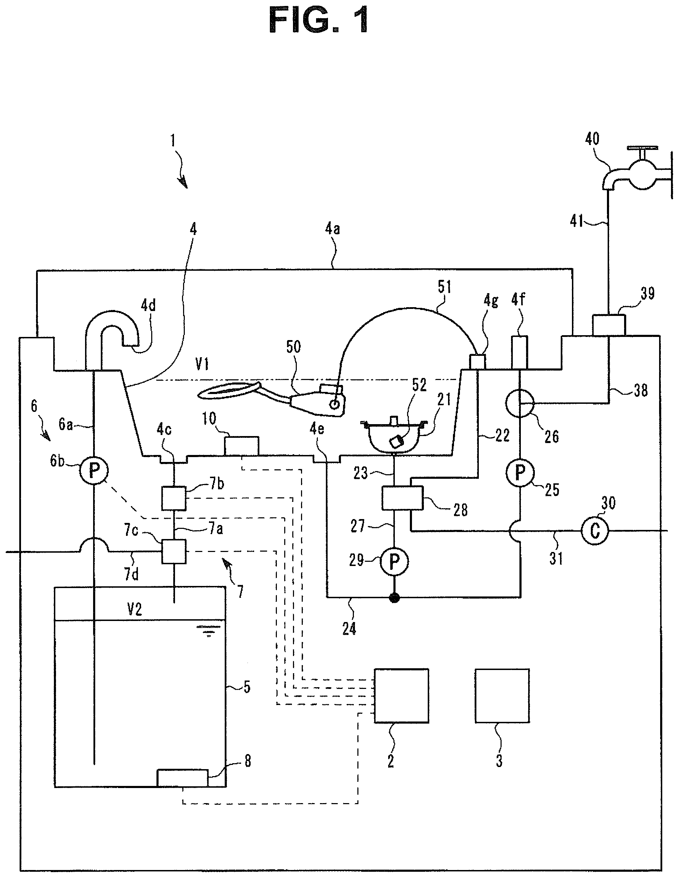

As shown in Fig. 1, the endoscope reprocessor 1 includes a control unit 2, a power supply apparatus 3, a treatment tank 4, a medicinal solution tank 5, a first transfer section 6, a second transfer section 7, a heating section 8 and a treatment tank temperature measuring section 10.

The control unit 2 is an apparatus configured to control actions of respective components which will be described later of the endoscope reprocessor 1 on the basis of a predetermined program, and includes a computer that is configured by including an arithmetic operation device, a storage device, an auxiliary storage device, an input/output device and the like. Control of the control unit 2 will be described later.

The power supply apparatus 3 is configured to supply electric power to the control unit 2 and the other components of the endoscope reprocessor 1. The power supply apparatus 3 may be a component that supplies electric power which is obtained from an outside such as a commercial power supply to the respective components, or may be a component that generates electric power for itself such as a primary battery, a secondary battery or a power generation apparatus.

The treatment tank 4 is a tub-shaped member having an opening portion that opens toward an upper part, as one example in the present embodiment, and the endoscope 50 can be disposed inside. At an upper portion of the treatment tank 4, a lid member 4a which opens and closes the opening portion is provided.

The treatment tank 4 has a capacity capable of storing a liquid of at least a predetermined volume V1 inside. The predetermined volume V1 is a value at which a whole of the endoscope 50 which is disposed in the treatment tank 4 can be submerged in the liquid, for example. Note that the capacity of the treatment tank 4 may be capable of storing a liquid of a larger volume than the above described predetermined volume V1. Though not illustrated, in the treatment tank 4, a water level sensor which detects whether or not a volume of a liquid which is stored is larger than the predetermined volume V1 is provided, and the control unit 2 controls the volume of the liquid which is stored in the treatment tank 4 on the basis of the detection result by the aforementioned water level sensor.

A medicinal solution introduction port 4d, a drainage port 4c and the treatment tank temperature measuring section 10 are provided in the treatment tank 4. The medicinal solution introduction port 4d is an opening portion which communicates with the first transfer section 6 which will be described later. The medicinal solution introduction port 4d is disposed at an upper part from a liquid level in a case where a liquid of the predetermined volume V1 is stored in the treatment tank 4. The drainage port 4c is an opening portion which opens in a bottom surface or a vicinity of the bottom surface of the treatment tank 4. The drainage port 4c communicates with the second transfer section 7 which will be described later.

The treatment tank temperature measuring section 10 measures a temperature of a liquid which is stored in the treatment tank 4. The treatment tank temperature measuring section 10 is electrically connected to the control unit 2, and a measurement result by the treatment tank temperature measuring section 10 is inputted to the control unit 2.

Further, in the treatment tank 4, a circulation port 4e, a circulation nozzle 4f, a connector section 4g and an accessory case 21 are provided.

The circulation port 4e is an opening portion that opens in the bottom face or the vicinity of the bottom face of the treatment tank 4. The circulation nozzle 4f is an opening portion which opens in the treatment tank 4. The circulation port 4e and the circulation nozzle 4f communicate with each other via a circulation conduit 24.

A changeover valve 26 is placed between the circulation nozzle 4f and the circulation conduit 24. One end of a water introduction conduit 38 is connected to the changeover valve 26. Though not illustrated, the changeover valve 26 is connected to the control unit 2. The changeover valve 26 is a three-way valve that can switch a state where the circulation nozzle 4f and the circulation conduit 24 are caused to communicate with each other, and a state where the circulation nozzle 4f and the water introduction conduit 38 are caused to communicate with each other.

The other end of the water introduction conduit 38 is connected to a water introduction valve 39. The water introduction valve 39 communicates with a water tap 40 which ejects water that is a liquid via a hose 41. Though not illustrated, the water introduction valve 39 is connected to the control unit 2. When the water introduction valve 39 is brought into an open state, and the changeover valve 26 is brought into a state where the circulation nozzle 4f and the water introduction conduit 38 are caused to communicate with each other, water is ejected from the circulation nozzle 4f and is introduced into the treatment tank 4.

The circulation conduit 24 is provided with a circulation pump 25. Though not illustrated, the circulation pump 25 is connected to the control unit 2. The changeover valve 26 is brought into the state where the circulation conduit 24 and the circulation nozzle 4f are caused to communicate with each other, and the circulation pump 25 is operated, whereby the liquid in the treatment tank 4 returns into the treatment tank 4 via the circulation conduit 24 and the circulation nozzle 4f after the liquid is sucked out from the circulation port 4e. That is, a flow occurs to the liquid which is stored in the treatment tank 4 by the operation of the circulation pump 25.

The connector section 4g is a portion that communicates with a conduit of the endoscope 50 via a connection tube 51, and introduces a fluid such as a liquid and air to insides of the conduit and the like of the endoscope 50. One end of a connector conduit 22 communicates with the connector section 4g. The connector section 4g communicates with a liquid feeding pump 29 and an air compressor 30 which will be described later via the connector conduit 22.

The accessory case 21 has a space that accommodates the accessory 52 which is taken out of the endoscope 50 inside the accessory case 21. The accessory 52 refers to a suction button, an air/water feeding button, a forceps plug or the like. One end of a case conduit 23 communicates with an inside of the accessory case 21. The accessory case 21 communicates with the liquid feeding pump 29 and the air compressor 30 which will be described later via the case conduit 23.

The other ends of the connector conduit 22 and the case conduit 23 are connected to a switching section 28. Further, one ends of a branch conduit 27 and an air feeding conduit 31 are connected to the switching section 28. The other end of the branch conduit 27 communicates with a segment between the circulation port 4e of the circulation conduit 24 and the circulation pump 25. The other end of the air feeding conduit 31 is opened to the atmosphere. Note that an air filter may be provided at the other end of the air feeding conduit 31.

The branch conduit 27 is provided with the liquid feeding pump 29. Though not illustrated, the liquid feeding pump 29 is connected to the control unit 2. The liquid feeding pump 29 is operated in a state where a liquid is stored in the treatment tank 4, whereby the liquid which is stored in the treatment tank 4 is transferred to the switching section 28 via the circulation port 4e, the circulation conduit 24 and the branch conduit 27.

The air feeding conduit 31 is provided with the air compressor 30. Though not illustrated, the air compressor 30 is connected to the control unit 2. By the air compressor 30 being operated, air is transferred to the switching section 28 via the air feeding conduit 31.

The switching section 28 switches connection states of the branch conduit 27 and the air feeding conduit 31, and the connector conduit 22 and the case conduit 23. Though not illustrated, the switching section 28 is connected to the control unit 2. The switching section 28 switches a state where the branch conduit 27 and the air feeding conduit 31 communicate with the connector conduit 22, and a state where the branch conduit 27 and the air feeding conduit 31 communicate with the case conduit 23. In accordance with a switching action of the switching section 28, fluids which are delivered from the liquid feeding pump 29 and the air compressor 30 are introduced into either one of the connector section 4g and the accessory case 21.

Note that the switching section 28 may have a configuration in which an alcohol feeding section that delivers an alcohol aqueous solution is connected to the switching section 28, and the switching section 28 causes the alcohol feeding section to communicate with either one of the connector conduit 22 and the case conduit 23.

The medicinal solution tank 5 stores a medicinal solution in a liquid state. A kind of medicinal solution which is stored in the medicinal solution tank 5 may be a sterilizing liquid, a disinfecting liquid, a cleaning liquid or the like, and is not specially limited. In the present embodiment, a disinfecting liquid that is a medicinal solution is stored in the medicinal solution tank 5, as an example.

The medicinal solution tank 5 has a capacity that stores a medicinal solution of a volume V2 which is larger than the aforementioned predetermined volume V1 of the liquid which can be stored in the treatment tank 4. Note that though not illustrated, a water level sensor that detects whether the volume of a medicinal solution which is stored is larger than the predetermined volume V2 is provided in the medicinal solution tank 5. The medicinal solution tank 5 is disposed at a lower part from the treatment tank 4. The medicinal solution tank 5 is connected to the treatment tank 4 via the first transfer section 6 and the second transfer section 7 which will be described later.

The heating section 8 is placed in the medicinal solution tank 5. The heating section 8 heats the medicinal solution in the medicinal solution tank 5. The heating section 8 heats a medicinal solution in the medicinal solution tank 5 when a temperature of the medicinal solution in the medicinal solution tank 5 is lower than a predetermined temperature T1, and thereby keeps the temperature of the medicinal solution in the medicinal solution tank 5 at the predetermined temperature T1 or more. The predetermined temperature T1 is a value within a temperature range in which a disinfecting effect of the medicinal solution is sufficiently exhibited when the medicinal solution is a disinfecting liquid, for example.

The first transfer section 6 transfers the medicinal solution stored in the medicinal solution tank 5 to the treatment tank 4. The first transfer section 6 includes a first conduit 6a and a medicinal solution pump 6b. The first conduit 6a is a conduit that causes the medicinal solution introduction port 4d and an inside of the medicinal solution tank 5 to communicate with each other.

The medicinal solution pump 6b is provided in the first conduit 6a. The medicinal solution pump 6b transfers the medicinal solution toward the medicinal solution introduction port 4d from the medicinal solution tank 5. The medicinal solution pump 6b is connected to the control unit 2. By the medicinal solution pump 6b being operated, the medicinal solution stored in the medicinal solution tank 5 is introduced into the treatment tank 4 through the medicinal solution introduction port 4d.

The second transfer section 7 transfers the medicinal solution stored in the treatment tank 4 to the medicinal solution tank 5. The second transfer section 7 includes a second conduit 7a and an opening and closing valve 7b. The second conduit 7a is a conduit configured to cause the drainage port 4c and the inside of the medicinal solution tank 5 to communicate with each other. The opening and closing valve 7b is an electromagnetic opening and closing valve configured to open and close the second conduit 7a. The opening and closing valve 7b is connected to the control unit 2.

Further, one end of a discharge conduit 7d is connected to a side of the second conduit 7a, which is nearer to the medicinal solution tank 5 than the opening and closing valve 7b. The other end of the discharge conduit 7d is connected to drainage equipment outside the endoscope reprocessor 1, for example. In a branch portion of the second conduit 7a and the discharge conduit 7d, a three-way valve 7c is provided. The three-way valve 7c switches a state where the second conduit 7a causes the drainage port 4c and the medicinal solution tank 5 to communicate with each other, and a state where the second conduit 7a causes the drainage port 4c and the discharge conduit 7d to communicate with each other. The three-way valve 7c is connected to the control unit 2. Note that a pump may be provided in at least one of the second conduit 7a and the discharge conduit 7d.

When the opening and closing valve 7b is brought into a closed state, and a liquid such as tap water and a medicinal solution is introduced into the treatment tank 4, the liquid is stored in the treatment tank 4. Further, when the opening and closing valve 7b is brought into an open state, and the three-way valve 7c is brought into a state where the second conduit 7a causes the drainage port 4c and the medicinal solution tank 5 to communicate with each other, the medicinal solution which is stored in the treatment tank 4 is transferred into the medicinal solution tank 5 by the gravity. Further, when the opening and closing valve 7b is brought into the open state, and the three-way valve 7c is brought into a state where the second conduit 7a causes the drainage port 4c and the discharge conduit 7d to communicate with each other, the liquid which is stored in the treatment tank 4 is discharged to an outside of the endoscope reprocessor 1 via the discharge conduit 7d.

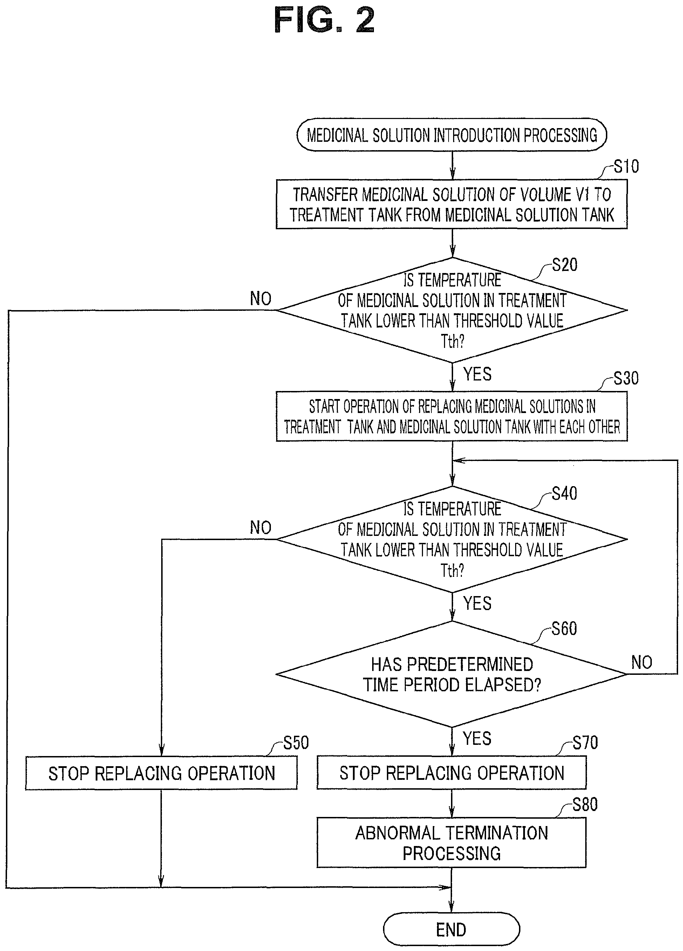

Next, control of the endoscope reprocessor 1 by the control unit 2 will be described. Fig. 2 is a flowchart of medicinal solution introduction processing of introducing a medicinal solution into the treatment tank 4 from the medicinal solution tank 5, in the endoscope reprocessor 1. The medicinal solution introduction processing is executed when disinfecting treatment is applied to an endoscope, when the medicinal solution is a disinfecting liquid, for example. Note that at a point of time of start of the medicinal solution introduction processing, the inside of the treatment tank 4 is in a state storing no liquid, and the medicinal solution tank 5 is in a state storing a medicinal solution of the predetermined volume V2. Further, at the point of time of start of the medicinal solution introduction processing, the three-way valve 7c is in a state where the second conduit 7a causes the drainage port 4c and the medicinal solution tank 5 to communicate with each other.

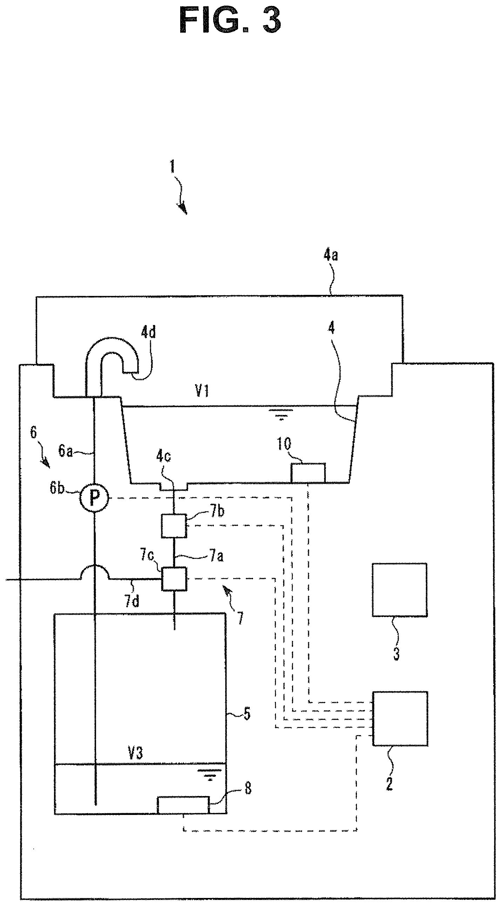

In the medicinal solution introduction processing, first of all, in step S10, a medicinal solution of the predetermined volume V1 is transferred into the treatment tank 4 from the medicinal solution tank 5. That is, after the opening and closing valve 7b is brought into a closed state, the first transfer section 6 is controlled to operate the medicinal solution pump 6b, and the medicinal solution of the predetermined volume V1 is transferred into the treatment tank 4 from the medicinal solution tank 5. By execution of step S10, the treatment tank 4 is brought into a state storing the medicinal solution of the predetermined volume V1, and the medicinal solution tank 5 is brought into a state where the medical liquid of a volume V3 obtained by the volume V1 being subtracted from a body fluid V2 remains, as shown in Fig. 3. Note that in Fig. 3, illustrations of the components which are not used in the medicinal solution introduction processing, the endoscope 50 and the like are omitted.

Next, in step S20, it is determined whether or not a measurement result by the treatment tank temperature measuring section 10 is lower than a predetermined threshold value Tth. That is, it is determined whether or not the temperature of the medicinal solution which is stored in the treatment tank 4 is lower than the predetermined threshold value Tth. The threshold value Tth is a value which is the aforementioned predetermined temperature T1 or less, and is a value within the temperature range where the disinfecting effect of the medicinal solution is sufficiently exhibited when the medicinal solution is a disinfecting solution, for example.

When it is determined that the measurement result by the treatment tank temperature measuring section 10 is the predetermined threshold value Tth or more in step S20, the medicinal solution introduction processing is normally terminated. When the determination (NO in step S20) is made, the temperature of the medicinal solution which is stored in the treatment tank 4 is within the range in which the medicinal solution sufficiently exhibits the effect of the medicinal solution, and the treatment using the medicinal solution can be subsequently executed in the treatment tank 4 in a preferable state.

When it is determined that the measurement result by the treatment tank temperature measuring section 10 is lower than the predetermined threshold value Tth in step S20, the flow shifts to step S30. In step S30, the first transfer section 6 and the second transfer section 7 are controlled, and an action of replacing at least a part of the medicinal solution stored in the treatment tank 4 with the medicinal solution which remains in the medicinal solution tank 5 is started.

A control mode of transfer of the medicinal solution by the first transfer section 6 and the second transfer section 7 in step S30 is not specially limited as long as at least a part of the medicinal solution stored in the treatment tank 4 is replaced with the medicinal solution which remains in the medicinal solution tank 5 in a predetermined time period under a condition that the medicinal solution does not overflow from the treatment tank 4. A time period in which the medicinal solution pump 6b of the first transfer section 6 is operated, and a time period in which the opening and closing valve 7b of the second transfer section 7 is brought into an open state may coincide with each other or does not have to coincide with each other. In other words, a time period in which the medicinal solution is transferred to the medicinal solution tank 5 from the treatment tank 4 and a time period in which the medicinal solution is transferred to the treatment tank 4 from the medicinal solution tank 5 may coincide with each other or does not have to coincide with each other.

That is, in step S30, control may be performed, which alternately repeats an action of transferring the medicinal solution of a predetermined volume V4 to the treatment tank 4 from the medicinal solution tank 5, and an action of transferring the medicinal solution of the aforementioned predetermined volume V4 to the medicinal solution tank 5 from the treatment tank 4. Further, in step S30, control may performed, which simultaneously performs an action of transferring the medicinal solution to the treatment tank 4 from the medicinal solution tank 5 at a predetermined flow rate Q, and an action of transferring the medicinal solution to the medicinal solution tank 5 from the treatment tank 4 at the aforementioned predetermined flow rate Q, and circulates the medicinal solution between the medicinal solution tank 5 and the treatment tank 4.

By execution of step S30, the medicinal solution with the temperature T1 that is the threshold value Tth or more, which remains in the medicinal solution tank 5 is transferred into the treatment tank 4, and the medicinal solution with a temperature which is lower than the threshold value Tth in the treatment tank 4 is transferred into the medicinal solution tank 5, so that the temperature of the medicinal solution which is stored in the treatment tank 4 starts to increase. Note that in step S30, an action of heating the medicinal solution in the medicinal solution tank 5 may be executed by controlling the heating section 8, simultaneously with the action of replacing the medicinal solution in the treatment tank 4 and the medicinal solution in the medicinal solution tank 5 with each other.

Next, in step S40, it is determined whether or not the measurement result by the treatment tank temperature measuring section 10 is lower than the predetermined threshold value Tth. That is, it is determined whether or not the temperature of the medicinal solution which is stored in the treatment tank 4 is lower than the predetermined threshold value Tth.

When it is determined that the measurement result by the treatment tank temperature measuring section 10 is the predetermined threshold value Tth or more in step S40, the flow shifts to step S50. In step S50, the action of replacing the medicinal solution in the treatment tank 4 and the medicinal solution in the medicinal solution tank 5 with each other, which is started in step S30 is stopped. Subsequently, the medicinal solution introduction processing is normally terminated.

When it is determined that the measurement result by the treatment tank temperature measuring section 10 is lower than the predetermined threshold value Tth in step S40, the flow shifts to step S60. In step S60, it is determined whether or not a predetermined time period elapses from start of the execution of step S30.

When it is determined that the predetermined time period does not elapse from the start of the execution of step S30 in step S60, the flow returns to step S40.

That is, when the temperature of the medicinal solution stored in the treatment tank 4 becomes the predetermined threshold value Tth or more within the predetermined time period after the action of replacing at least a part of the medicinal solution stored in the treatment tank 4 with the medicinal solution which remains in the medicinal solution tank 5 is started in step S30, the medicinal solution introduction processing is normally terminated. In this case, the temperature of the medicinal solution which is stored in the treatment tank 4 is within the range in which the effect is sufficiently exhibited, and treatment using the medicinal solution can be subsequently executed in the treatment tank 4 in the preferable state.

When it is determined that the predetermined time period elapses from the start of execution of step S30, in step S60, the flow shifts to step S70. In step S70, the action of replacing the medicinal solution in the treatment tank 4 and the medicinal solution in the medicinal solution tank 5 with each other, which is started in step S30, is stopped. Subsequently, the flow shifts to step S80, and abnormal termination processing is executed. In the abnormal termination processing in step S80, the medicinal solution in the treatment tank 4 is all transferred into the medicinal solution tank 5, and the medicinal solution is heated again by the heating section 8, for example. Note that in the abnormal termination processing in step S80, a user may be notified that the temperature of the medicinal solution stored in the treatment tank 4 does not become the threshold value Tth or more, by output of a warning sound, output of an alarm display or the like.

In this way, when the temperature of the medicinal solution stored in the treatment tank 4 does not become the predetermined threshold value Tth or more within the predetermined time period after the action of replacing at least a part of the medicinal solution stored in the treatment tank 4 with the medicinal solution which remains in the medicinal solution tank 5 is started in step S30, the medicinal solution introduction processing is abnormally terminated.

As described above, the endoscope reprocessor 1 in the present embodiment includes the treatment tank 4 configured to accommodate the endoscope 50 and cause the endoscope 50 to contact the medicinal solution, the treatment tank temperature measuring section 10 configured to measure the temperature of the medicinal solution stored in the treatment tank 4, the medicinal solution tank 5 configured to have the larger storage capacity for the medicinal solution than the treatment tank 4, the heating section 8 configured to heat the medicinal solution in the medicinal solution tank 5, the first transfer section 6 configured to transfer a part of the medicinal solution heated by the heating section 8 to the treatment tank 4 from the medicinal solution tank, the second transfer section 7 configured to transfer the medicinal solution in the treatment tank 4 to the medicinal solution tank 5, and the control unit 2 configured to be connected to the treatment tank temperature measuring section 10, the first transfer section 6 and the second transfer section 7. The control unit 2 replaces at least a part of the medicinal solution stored in the treatment tank 4 with the medicinal solution which remains in the medicinal solution tank 5 by controlling the first transfer section 6 and the second transfer section 7, when the measurement result by the treatment tank temperature measuring section 10 is lower than the predetermined threshold value Tth, in the medicinal solution introduction processing of introducing the medicinal solution into the treatment tank 4 from the medicinal solution tank 5.

Since in the endoscope reprocessor 1 of the present embodiment, the temperature of the medicinal solution stored in the treatment tank 4 is increased immediately by at least parts of the medicinal solutions in the treatment tank 4 and the medicinal solution tank 5 being replaced with each other, the medicinal solution does not have to be reheated by the heating section 8, and a time required to carry out reprocessing can be reduced.

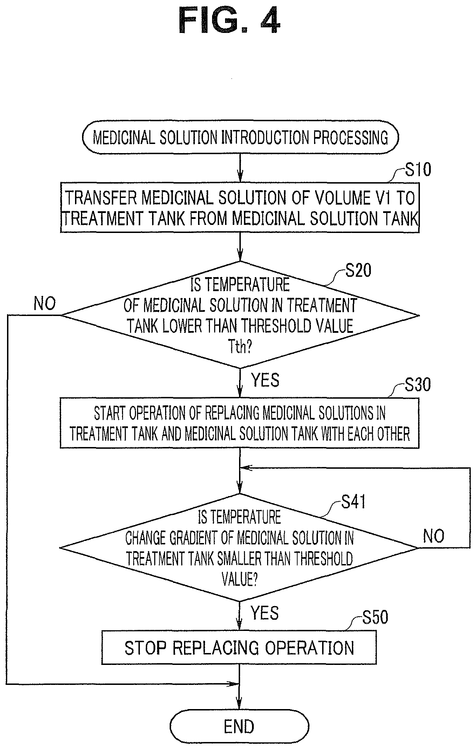

Fig. 4 shows a modification of the endoscope reprocessor 1 of the present embodiment. Fig. 4 is a flowchart showing a modification of the medicinal solution introduction processing of the endoscope reprocessor 1 of the first embodiment.

In the aforementioned first embodiment, the action of replacing the medicinal solution in the treatment tank 4 and the medicinal solution in the medicinal solution tank 5 with each other is started by execution of step S30, and thereafter, when the temperature of the medicinal solution stored in the treatment tank 4 becomes the threshold value Tth or more, as shown in step S40, the aforementioned replacing action is stopped.

In the present modification, the action of replacing the medicinal solution in the treatment tank 4 and the medicinal solution in the medicinal solution tank 5 with each other is started by execution of step S30, and thereafter, when a temperature change gradient of the medicinal solution stored in the treatment tank 4 becomes smaller than a predetermined threshold value, the aforementioned replacing action is stopped, as shown in step S41 in Fig. 4. Here, the temperature change gradient of the medicinal solution stored in the treatment tank 4 refers to a temperature change amount per unit time of the measurement result by the treatment tank temperature measuring section 10.

That is, in the present modification, the action of replacing the medicinal solution in the treatment tank 4 and the medicinal solution in the medicinal solution tank 5 with each other, which is started in step S30 is stopped when the temperature change of the medicinal solution stored in the treatment tank 4 becomes smaller than a value set in advance, or the temperature change becomes nonexistent.

Since in the endoscope reprocessor 1 of the present modification, the temperature of the medicinal solution stored in the treatment tank 4 is also immediately increased by at least parts of the medicinal solutions in the treatment tank 4 and the medicinal solution tank 5 being replaced with each other, there is no need to reheat the medicinal solution by the heating section 8, and a time period required to carry out reprocessing can be reduced.

Step S60 of confirming the lapse of the predetermined time period and step S80 of abnormal termination processing which are shown in Fig. 2 may be added to the present step. In this case, S60 is interposed in a middle of a step of returning to S30 from S41, and in a case of NO in S60, the flow goes to S30, whereas in a case of YES in S60, the flow goes to S80.

### (Second embodiment)

Next, a second embodiment of the present invention will be described. Hereinafter, only a difference from the first embodiment will be described, the same reference signs will be assigned to the similar components to the components in the first embodiment, and explanation of the similar components will be properly omitted.

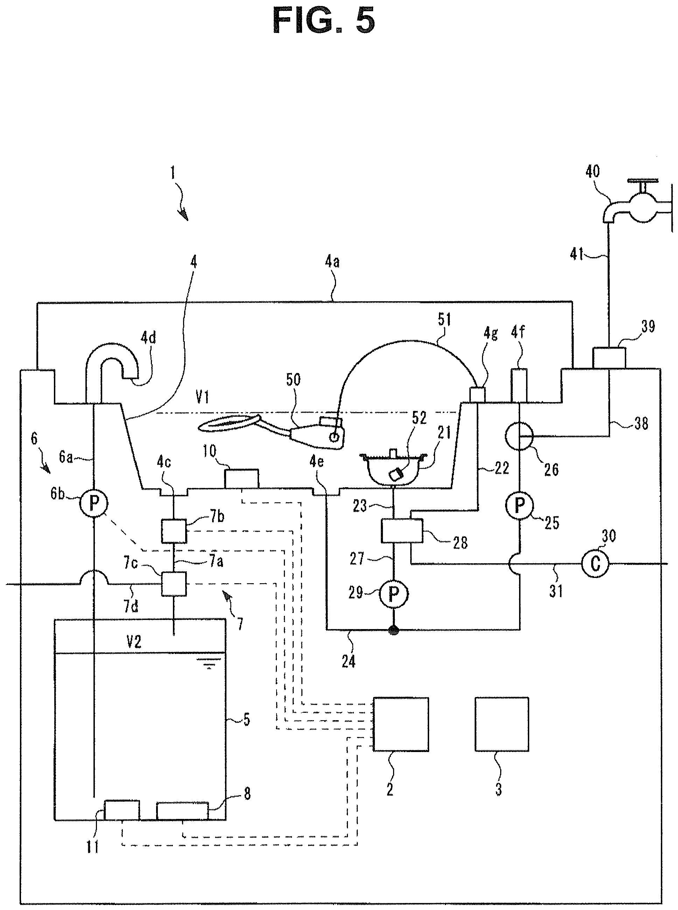

The endoscope reprocessor 1 of the present embodiment shown in Fig. 5 differs from the first embodiment in a point in which the endoscope reprocessor 1 of the present embodiment includes a medicinal solution tank temperature measuring section 11 in the medicinal solution tank 5. The medicinal solution tank temperature measuring section 11 measures the temperature of the medicinal solution which is stored in the medicinal solution tank 5. The medicinal solution tank temperature measuring section 11 is electrically connected to the control unit 2, and a measurement result by the medicinal solution tank temperature measuring section 11 is inputted to the control unit 2.

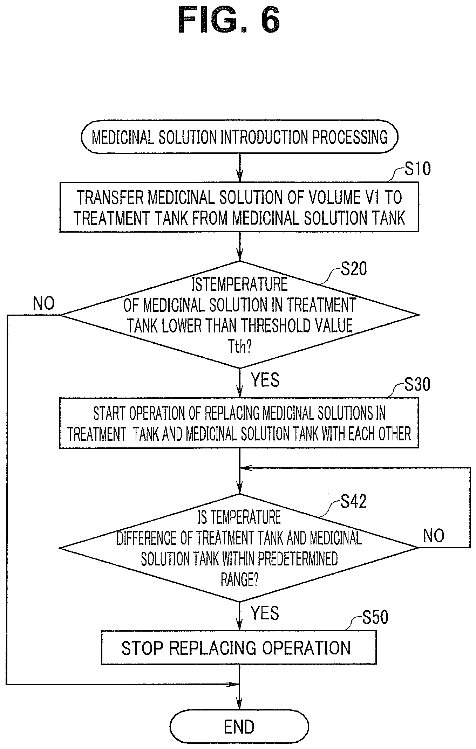

Fig. 6 is a flowchart of medicinal solution introduction processing of the endoscope reprocessor 1 of the second embodiment.

In the present embodiment, when the action of replacing the medicinal solution in the treatment tank 4 and the medicinal solution in the medicinal solution tank 5 with each other is started by execution of step S30, and thereafter, a difference between the measurement result by the treatment tank temperature measuring section 10 and the measurement result by the medicinal solution tank temperature measuring section 11 stays within a predetermined range, as shown in step S42 in Fig. 6, the aforementioned replacing action is stopped.

Since in the endoscope reprocessor 1 of the present embodiment, the temperature of the medicinal solution which is stored in the treatment tank 4 is also immediately increased by at least parts of the medicinal solutions in the treatment tank 4 and in the medicinal solution tank 5 being replaced with each other, there is no need to reheat the medicinal solution by the heating section 8, and a time period required to carry out reprocessing can be reduced.

Step S60 of confirming the lapse of the predetermined time period and step S80 of abnormal termination processing which are shown in Fig. 2 may be added to the present step. In this case, S60 is interposed in a middle of a step of returning to S30 from S42, and in a case of NO in S60, the flow goes to S30, whereas in a case of YES in S60, the flow goes to S80.

### (Third embodiment)

Next, a third embodiment of the present invention will be described. Hereinafter, only a difference from the second embodiment will be described, the same reference signs will be assigned to the similar components to the components in the second embodiment, and explanation of the similar components will be properly omitted.

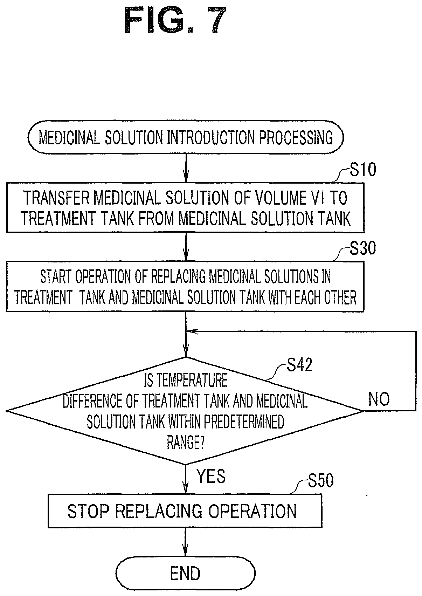

Fig. 7 is a flowchart of medicinal solution introduction processing of the endoscope reprocessor 1 of the third embodiment.

In the medicinal solution introduction processing of the endoscope reprocessor 1 of the aforementioned second embodiment, determination of whether or not the measurement result by the treatment tank temperature measuring section 10 is lower than the predetermined threshold value is performed (step S20), and when the measurement result by the treatment tank temperature measuring section 10 is lower than the predetermined threshold value, the action of replacing the medicinal solution in the treatment tank 4 and the medicinal solution in the medicinal solution tank 5 with each other in step S30 and the following step is executed.

In the endoscope reprocessor 1 of the present embodiment differs from the second embodiment in a point in which determination of whether or not the measurement result by the treatment tank temperature measuring section 10 is lower than the predetermined threshold value is not performed, as shown in Fig. 7.

That is, in the medicinal solution introduction processing in the present embodiment, the medicinal solution of the predetermined volume V1 is transferred into the treatment tank 4 from the medicinal solution tank 5 first in step S10.

Next, in step S30, the first transfer section 6 and the second transfer section 7 are controlled, and an action of replacing at least a part of the medicinal solution stored in the treatment tank 4 with the medicinal solution which remains in the medicinal solution tank 5 is started.

Since the medicinal solution circulates between the medicinal solution tank 5 and the treatment tank 4 by execution of step S30, the temperatures of the treatment tank 4 and a spot that contacts the medicinal solution, of the first transfer section 6 approach the temperature T1 of the medicinal solution which is stored in the medicinal solution tank 5.

For example, when step S10 is executed in a case where a temperature of a wall face of the treatment tank 4 is the temperature T1 or less in a case where an outside temperature is low or the like, the temperature of the medicinal solution which is stored in the treatment tank 4 becomes lower than the temperature T1. However, since the medicinal solution with the temperature T1 which remains in the medicinal solution tank 5 circulates between the medicinal solution tank 5 and the treatment tank 4 by step S30 being executed, the temperature of the medicinal solution which is stored in the treatment tank 4 starts to increase. Note that in step S30, an action of controlling the heating section 8 and heating the medicinal solution in the medicinal solution tank 5 may be executed simultaneously with the action of replacing the medicinal solution in the treatment tank 4 and the medicinal solution in the medicinal solution tank 5 with each other.

Next, when the difference between the measurement result by the treatment tank temperature measuring section 10 and the measurement result by the medicinal solution tank temperature measuring section 11 stays within the predetermined range, after the action of replacing the medicinal solution in the treatment tank 4 and the medicinal solution in the medicinal solution tank 5 with each other is started by execution of step S30, as shown in step S42, the aforementioned replacing action is stopped.

Since in the endoscope reprocessor 1 of the present embodiment, the temperature of the medicinal solution which is stored in the treatment tank 4 is also immediately increased by at least a part of the medicinal solution in the treatment tank 4 and a part of the medicinal solution in the medicinal solution tank 5 being replaced with each other, there is no need to reheat the medicinal solution by the heating section 8, and a time period required to carry out reprocessing can be reduced.

Step S60 of confirming the lapse of the predetermined time period and step S80 of abnormal termination processing which are shown in Fig. 2 may be added to the present step. In this case, S60 is interposed in a middle of a step of returning to S30 from S42, and in a case of NO in S60, the flow goes to S30, whereas in a case of YES in S60, the flow goes to S80.

### (Fourth embodiment)

Next, a fourth embodiment of the present invention will be described. Hereinafter, only a difference from the first embodiment will be described, the same reference signs will be assigned to the similar components to the components in the first embodiment, and explanation of the similar components will be properly omitted.

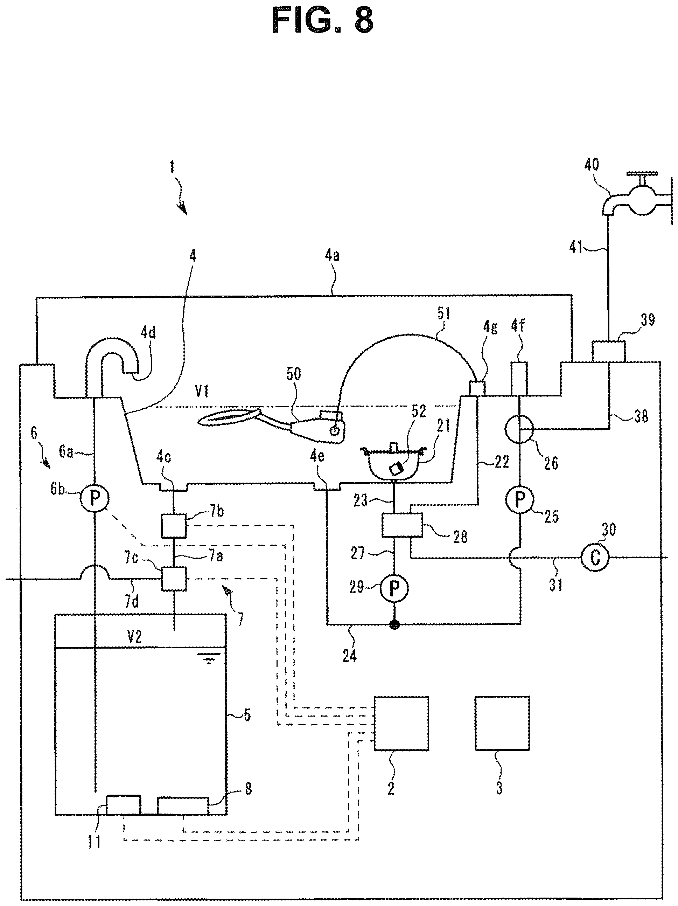

The endoscope reprocessor 1 of the present embodiment shown in Fig. 8 differs from the first embodiment in a point in which the endoscope reprocessor 1 of the present embodiment includes a medicinal solution tank temperature measuring section 11 in the medicinal solution tank 5. The medicinal solution tank temperature measuring section 11 measures the temperature of the medicinal solution which is stored in the medicinal solution tank 5. The medicinal solution tank temperature measuring section 11 is electrically connected to the control unit 2, and a measurement result by the medicinal solution tank temperature measuring section 11 is inputted to the control unit 2.

Note that although not illustrated in Fig. 8, the endoscope reprocessor 1 of the present embodiment may include the treatment tank temperature measuring section 10 treatment tank temperature measuring section that measures the temperature of the medicinal solution which is stored in the treatment tank 4.

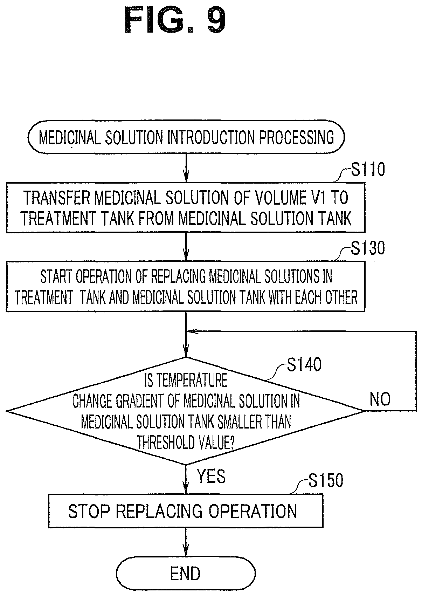

Fig. 9 is a flowchart of medicinal solution introduction processing of the endoscope reprocessor 1 of the third embodiment.

In the medicinal solution introduction processing in the endoscope reprocessor 1 of the third present embodiment, the medicinal solution of the predetermined volume V1 is transferred into the treatment tank 4 from the medicinal solution tank 5 first in step S110. That is, after the opening and closing valve 7b is brought into a closed state, the first transfer section 6 is controlled to operate the medicinal solution pump 6b, and the medicinal solution of the predetermined volume V1 is transferred into the treatment tank 4 from the medicinal solution tank 5. By execution of step S110, the treatment tank 4 is brought into a state where the medicinal solution of the predetermined volume V1 is stored, and the medicinal solution tank 5 is brought into a state where the medicinal solution of a volume V3 obtained by the volume V1 being subtracted from a body fluid V2.

Next, in step S130, the first transfer section 6 and the second transfer section 7 are controlled, and an action of replacing at least a part of the medicinal solution stored in the treatment tank 4 with the medicinal solution which remains in the medicinal solution tank 5 is started.

A control mode of transfer of the medicinal solution by the first transfer section 6 and the second transfer section 7 in step S130 is not specially limited as long as at least a part of the medicinal solution stored in the treatment tank 4 is replaced with the medicinal solution which remains in the medicinal solution tank 5 in a predetermined time period, under a condition that the medicinal solution does not overflow from the treatment tank 4. A time period in which the medicinal solution pump 6b of the first transfer section 6 is operated, and a time period in which the opening and closing valve 7b of the second transfer section 7 is brought into an open state may coincide with each other or do not have to coincide with each other. In other words, a time period in which the medicinal solution is transferred to the medicinal solution tank 5 from the treatment tank 4 and a time period in which the medicinal solution is transferred to the treatment tank 4 from the medicinal solution tank 5 may coincide with each other or does not have to coincide with each other.

That is, in step S130, control may be performed, which alternately repeats an action of transferring the medicinal solution of a predetermined volume V4 to the treatment tank 4 from the medicinal solution tank 5, and an action of transferring the medicinal solution of the aforementioned predetermined volume V4 to the medicinal solution tank 5 from the treatment tank 4. Further, in step S130, control may be performed, which simultaneously performs an action of transferring the medicinal solution to the treatment tank 4 from the medicinal solution tank 5 at a predetermined flow rate Q, and an action of transferring the medicinal solution to the medicinal solution tank 5 from the treatment tank 4 at the aforementioned predetermined flow rate Q, and causes the medicinal solution to circulate between the medicinal solution tank 5 and the treatment tank 4.

Since the medicinal solution circulates between the medicinal solution tank 5 and the treatment tank 4 by execution of step S130, the temperature of the treatment tank 4 and a spot that contacts the medicinal solution, of the first transfer section 6 approaches the temperature T1 of the medicinal solution stored in the medicinal solution tank 5.

For example, when step S110 is executed in a case where a temperature of a wall face of the treatment tank 4 is the temperature T1 or less in a case where an outside temperature is low or the like, the temperature of the medicinal solution which is stored in the treatment tank 4 becomes lower than the temperature T1. However, since the medicinal solution with the temperature T1 which remains in the medicinal solution tank 5 circulates between the medicinal solution tank 5 and the treatment tank 4 by step S130 being executed, the temperature of the medicinal solution which is stored in the treatment tank 4 starts to increase. Note that in step S130, an action of controlling the heating section 8 and heating the medicinal solution in the medicinal solution tank 5 may be executed simultaneously with the action of replacing the medicinal solution in the treatment tank 4 and the medicinal solution in the medicinal solution tank 5 with each other.

Next, in step S140, it is determined whether or not a temperature change gradient of the medicinal solution in the medicinal solution tank 5 becomes smaller than a predetermined threshold value. Here, the temperature change gradient of the medicinal solution in the medicinal solution tank 5 refers to a temperature change amount per unit time of the measurement result by the medicinal solution tank temperature measuring section 11.

In step S140, the action of replacing the medicinal solution in the treatment tank 4 and the medicinal solution in the medicinal solution tank 5 with each other is continued, until the temperature change gradient of the medicinal solution in the medicinal solution tank 5 becomes smaller than the predetermined threshold value. When it is determined that the temperature change gradient of the medicinal solution in the medicinal solution tank 5 becomes smaller than the predetermined threshold value in step S140, the flow shifts to step S150, and the action of replacing the medicinal solution in the treatment tank 4 and the medicinal solution in the medicinal solution tank 5 with each other is stopped. That is, in the present embodiment, the action of replacing the medicinal solution in the treatment tank 4 and the medicinal solution in the medicinal solution tank 5 with each other, which is started in step S130, is stopped when the temperature change of the medicinal solution in the medicinal solution tank 5 becomes smaller than a value set in advance, or the temperature change becomes nonexistent.

Since in the endoscope reprocessor 1 of the present embodiment, the temperature of the medicinal solution which is stored in the treatment tank 4 is also immediately increased by at least parts of the medicinal solutions in the treatment tank 4 and in the medicinal solution tank 5 being replaced with each other, there is no need to reheat the medicinal solution by the heating section 8, and a time period required to carry out reprocessing can be reduced.

Step S60 of confirming the lapse of the predetermined time period and step S80 of abnormal termination processing which are shown in Fig. 2 may be added to the present step. In this case, S60 is interposed in a middle of a step of returning to S130 from S140, and in a case of NO in S60, the flow goes to S30, whereas in a case of YES in S60, the flow goes to S80.

Note that the present invention is not limited to the aforementioned embodiments, and can be properly changed within the range without departing from the gist or the idea of the invention which is readable from the claims and entire specification, and endoscope reprocessors with the changes like this are also included in the technical range of the present invention.

The present application is field on the basis of claim of priority of Japanese Patent Application No. 2015-23439 filed in Japan on February 9, 2015, and the above described disclosed contents are incorporated in the description, the claims and the drawings of the present application by reference.

## Claims

1. An endoscope reprocessor, comprising:
a treatment tank configured to accommodate an endoscope to cause the endoscope to contact a medicinal solution;
a treatment tank temperature measuring section configured to measure a temperature of the medicinal solution stored in the treatment tank;
a medicinal solution tank configured to have a larger storage capacity for the medicinal solution than the treatment tank;
a heating section configured to heat the medicinal solution inside the medicinal solution tank;
a first transfer section configured to transfer a part of the medicinal solution heated by the heating section to the treatment tank from the medicinal solution tank;
a second transfer section configured to transfer the medicinal solution of the treatment tank to the medicinal solution tank; and
a control unit configured to be connected to the treatment tank temperature measuring section, the first transfer section and the second transfer section, and replace at least a part of the medicinal solution stored in the treatment tank with the medicinal solution remaining in the medicinal solution tank by controlling the first transfer section and the second transfer section, when a measurement result by the treatment tank temperature measuring section is lower than a predetermined threshold value.

2. The endoscope reprocessor according to claim 1,
wherein the control unit
causes the medicinal solution to circulate between the treatment tank and the medicinal solution tank by controlling the first transfer section and the second transfer section, until the measurement result by the treatment tank temperature measuring section does not change for a predetermined time period.

3. The endoscope reprocessor according to claim 1, further comprising:
a medicinal solution tank temperature measuring section configured to measure a temperature of the medicinal solution stored in the medicinal solution tank,
wherein the control unit
is connected to the medicinal solution tank temperature measuring section, and
causes the medicinal solution to circulate between the treatment tank and the medicinal solution tank by controlling the first transfer section and the second transfer section, until a temperature difference between the treatment tank and the medicinal solution tank stays within a predetermined range.

4. An endoscope reprocessor, comprising:
a treatment tank configured to accommodate an endoscope to cause the endoscope to contact a medicinal solution;
a medicinal solution tank configured to have a larger storage capacity for the medicinal solution than the treatment tank;
a medicinal solution tank temperature measuring section configured to measure a temperature of the medicinal solution stored in the medicinal solution tank;
a heating section configured to heat the medicinal solution inside the medicinal solution tank;
a first transfer section configured to transfer a part of the medicinal solution heated by the heating section to the treatment tank from the medicinal solution tank;
a second transfer section configured to transfer the medicinal solution of the treatment tank to the medicinal solution tank; and
a control unit configured to be connected to the medicinal solution tank temperature measuring section, the first transfer section and the second transfer section, and causes the medicinal solution to circulate between the treatment tank and the medicinal solution tank by controlling the first transfer section and the second transfer section, until a measurement result by the medicinal solution tank temperature measuring section does not change for a predetermined time period.

5. An endoscope reprocessor, comprising:
a treatment tank configured to accommodate an endoscope to cause the endoscope to contact a medicinal solution;
a treatment tank temperature measuring section configured to measure a temperature of the medicinal solution stored in the treatment tank;
a medicinal solution tank configured to have a larger storage capacity for the medicinal solution than the treatment tank;
a medicinal solution tank temperature measuring section configured to measure a temperature of the medicinal solution stored in the medicinal solution tank;
a heating section configured to heat the medicinal solution inside the medicinal solution tank;
a first transfer section configured to transfer a part of the medicinal solution heated by the heating section to the treatment tank from the medicinal solution tank;
a second transfer section configured to transfer the medicinal solution of the treatment tank to the medicinal solution tank; and
a control unit configured to be connected to the treatment tank temperature measuring section, the medicinal solution tank temperature measuring section, the first transfer section and the second transfer section, and causes the medicinal solution to circulate between the treatment tank and the medicinal solution tank by controlling the first transfer section and the second transfer section, until a temperature difference between the treatment tank and the medicinal solution tank stays in a predetermined range.
